# FASCICULE DE BREVET EUROPEEN

(11) **EP 3 478 851 B1**
(45) Date de publication et mention de la délivrance du brevet: **06.09.2023**
(21) Numéro de dépôt: 17723131.3
(22) Date de dépôt: 17.05.2017
(51) Int. Cl.: C12Q 1/04, G01N 33/18, C02F 1/00

(54) **PROCÉDÉ DE CONTRÔLE DE LA CONCENTRATION DE BACTÉRIES DANS UN RÉSEAU DE DISTRIBUTION D'EAU**
VERFAHREN ZUR ÜBERWACHUNG DER BAKTERIENKONZENTRATION IN EINEM WASSERVERTEILUNGSNETZ
PROCESS FOR MONITORING THE CONCENTRATION OF BACTERIA IN A WATER DISTRIBUTION NETWORK

(30) Priorité: 30.06.2016 FR 1656225
(43) Date de publication de la demande: 08.05.2019
(73) Titulaire: Suez International, 92040 Paris La Défense Cedex (FR)
(72) Inventeur: DO QUANG, Zdravka, 78870 Bailly (FR); COURTOIS, Sophie, 78230 Le Pecq (FR); CUSSONNEAU, Guillaume, 75009 Paris (FR); FAY, Gilles, 75019 Paris (FR)
(74) Mandataire: Atout PI Laplace
(86) Numéro de dépôt international: PCT/EP2017/061798
(87) Numéro de publication internationale: WO 2018/001627

(56) Documents cités:
- US-A1- 2004 020 862
- US-A1- 2004 158 432
- US-A1- 2005 009 192
- M BAKKER ET AL: "Monitoring water supply systems for anomaly detection and response", , 4 novembre 2012 (2012-11-04), XP055344872, Extrait de l'Internet: URL:http://repository.tudelft.nl/islandora /object/uuid:44c1e071-0cc6-4349-90e6-cb9b5 6eb994f/datastream/OBJ/download [extrait le 2017-02]
- FLOR RAMÍREZ-CASTILLO ET AL: "Waterborne Pathogens: Detection Methods and Challenges", PATHOGENS, vol. 4, no. 2, 21 mai 2015 (2015-05-21), pages 307-334, XP055345663, DOI: 10.3390/pathogens4020307
- DATABASE COMPENDEX [Online] ENGINEERING INFORMATION, INC., NEW YORK, NY, US; août 2010 (2010-08), FISHER S: "Free software tool to monitor water supply", XP055351285, Database accession no. E20103813246460 & POLLUTION ENGINEERING AUGUST 2010 BNP MEDIA USA, vol. 42, no. 8, août 2010 (2010-08),
- DATABASE COMPENDEX [Online] ENGINEERING INFORMATION, INC., NEW YORK, NY, US; 2010, FISHER S: "Free software tool to monitor water supply", Database accession no. E20103813246460 & FISHER S: "Free software tool to monitor water supply", POLLUTION ENGINEERING 2010 BNP MEDIA USA, vol. 42, no. 8, 2010,

## Description

L'invention se situe dans le domaine de la mesure de la qualité bactériologique en ligne sur des circuits de distribution d'eau et concerne un procédé de contrôle de la concentration de bactéries dans un réseau de distribution d'eau. L'invention concerne aussi un dispositif de contrôle de la concentration de bactéries dans un réseau de distribution d'eau. L'invention peut être appliquée au suivi de tout type d'eau, que ce soit des eaux de réseau de refroidissement, des eaux naturelles, des eaux environnementales ou des eaux traitées.

L'eau du robinet fait l'objet d'un suivi sanitaire permanent, destiné à en garantir la sécurité sanitaire, c'est-à-dire l'absence de micro-organismes pathogènes. En tant que micro-organismes pathogènes, on peut citer les bactéries, les virus et les parasites. Dans cette demande, nous parlerons seulement de bactéries mais il est bien entendu que l'invention s'applique de façon similaire au contrôle de la concentration de tout micro-organisme, qu'il soit pathogène ou non-pathogène.

La présence de bactéries dans les eaux de consommation est le plus souvent due à une dégradation de la qualité de la ressource en eau, à une mauvaise protection ou à un manque d'entretien des ouvrages de captage, à une défaillance du traitement de désinfection ou à une contamination de l'eau lors de son transport ou stockage dans le réseau.

Les méthodes pour rechercher les germes pathogènes dans l'eau sont longues et complexes, c'est pourquoi la qualité bactériologique de l'eau est généralement appréciée à partir de la recherche de germes témoins. Ainsi, la mise en évidence de ces germes dans l'eau témoigne de la possibilité de présence de germes pathogènes. De même, d'autres germes regroupant tous les micro-organismes pouvant croître sur un substrat nutritif non spécifique aux conditions standard témoignent de la charge globale en micro-organismes dans l'eau.

Cependant, ces indicateurs reposent tous sur des procédés de détection par culture. Ils nécessitent des prélèvements ponctuels, des analyses en laboratoire et des durées d'incubation allant de plusieurs heures à plusieurs jours. Par ailleurs, seuls les micro-organismes viables et cultivables peuvent être dénombrés, mais ils ne représentant qu'une faible proportion des germes présents dans l'eau. De plus, les contaminations microbiologiques sont susceptibles de se produire de façon sporadique sur de faibles intervalles de temps et leurs origines peuvent être très diverses. On peut notamment citer une défaillance ponctuelle du traitement, une remise en suspension des sédiments ou le décrochage du biofilm, des fuites, un retour d'eaux ou même du vandalisme. Ce type de méthode est ainsi peu fiable.

Il apparait donc nécessaire de faire un suivi en continu de la microflore de l'eau et d'effectuer des mesures en temps réel, avec par exemple une fréquence d'analyse inférieure à 30 minutes.

Il existe des méthodes de mesures en ligne. Les plus avancées pour le suivi de la qualité de l'eau utilisent des sondes multiparamétriques permettant de mesurer un grand nombre de paramètres physico-chimiques. Ces méthodes sont de mauvais indicateurs de la contamination microbiologique d'un liquide. Parmi eux, seule la turbidité peut être utilisée comme indicateur global de la qualité microbiologique mais elle peut aussi être affectée par la présence de diverses matières en suspension telles que le limon, l'argile, des matières organiques et inorganiques en fines particules et autres composés organiques colorés solubles.

Des méthodes optiques et en particulier le comptage des particules par reconnaissance de formes à partir d'images ou par diffusion ou occultation d'un faisceau de lumière peuvent être utilisées pour étudier la teneur en particules et/ou en bactéries ou micro-organismes. Aussi, des mesures par fluorescence sont également possibles. Ainsi, il existe des moyens de mesure en continu et en ligne, délivrant un signal en termes de nombre en particules ou en bactéries par unité de volume d'échantillon liquide analysé. Le signal obtenu est alors comparé à une valeur de seuil préalablement établie et une alarme peut être générée si le signal obtenu dépasse la valeur de seuil préétablie. Sur le même principe, l'alarme peut être générée selon l'évolution du signal, au-delà d'une vitesse d'augmentation du signal préétablie. L'établissement préalable de cette valeur de seuil, ou vitesse d'augmentation du signal, repose sur l'empirisme ou se fait par interprétation de la ligne de base qui nécessite de réaliser au préalable une série de mesures en continu de la concentration totale en bactéries sur une fenêtre temporelle d'une à plusieurs semaines. La valeur de seuil est donc bien souvent imprécise et ne correspond pas forcément aux conditions de fonctionnement du réseau de distribution. Le signal obtenu, par exemple du nombre de bactéries totales, est mal exploité. En effet, si une augmentation du nombre de bactéries totales est observée, ce signal n'est pas traité de façon à pouvoir différencier une augmentation due à un changement normal des conditions, par exemple suite à une modification du débit d'eau dans le réseau de distribution d'eau, ou à un changement anormal, par exemple un défaut au niveau du traitement de l'eau.

Le document US2005/0009192 décrit un système de détection de la qualité de l'eau dans un réseau de distribution. Des attributs de l'eau sont mesurés et comparés avec un signal représentatif corrélé enregistré dans une base de données et correspondant à une condition particulière de l'eau.

L'invention vise à pallier tout ou partie des problèmes cités plus haut en proposant un procédé de contrôle de la concentration de bactéries dans un réseau de distribution d'eau basé sur une valeur de seuil s'adaptant aux différentes données disponibles sur le réseau de distribution. Ce procédé permet d'améliorer l'utilisation opérationnelle des données issues du suivi de la bactériologie pour les réseaux d'eau. Le traitement des données permet ainsi à un opérateur de réseau une meilleure visualisation du comportement du réseau et le déclenchement des actions correctives et préventives avec une plus grande efficacité.

A cet effet, l'invention a pour objet un procédé de contrôle de la concentration de bactéries dans l'eau d'un réseau de distribution d'eau dans des conditions environnantes, caractérisé en ce qu'il comprend les étapes suivantes:
- mesure de la concentration de bactéries dans l'eau par un premier capteur positionné à une première localisation dans le réseau de distribution d'eau (étape 1001),
- détermination d'une unique valeur instantanée variable de la concentration de bactéries attendue dans l'eau à la première localisation, valeur évolutive et régulièrement mise à jour, en fonction d'un paramètre comprenant des caractéristiques physico-chimiques et/ou bactériologiques de l'eau, ledit paramètre étant évolutif et prenant en compte les conditions environnantes du réseau d'eau (étape 1002),
- à une fréquence prédéfinie : comparaison de la concentration de bactéries dans l'eau mesurée par le premier capteur avec la valeur instantanée variable (étape 1003).

Selon un mode de réalisation, le procédé de contrôle selon l'invention peut comprendre une action correctrice agissant sur la concentration de bactéries dans l'eau si la concentration de bactéries dans l'eau mesurée par le premier capteur est supérieure à la valeur instantanée variable.

Selon un mode de réalisation, le procédé de contrôle selon l'invention comprend une étape de sauvegarde de la concentration de bactéries dans l'eau mesurée par le premier capteur dans une base de données de façon à constituer un historique des concentrations de bactéries dans l'eau mesurées par le premier capteur, et la détermination du paramètre caractéristique de l'eau se fait en fonction de l'historique des concentrations de bactéries dans l'eau mesurées par le premier capteur.

Selon un autre mode de réalisation, le procédé de contrôle selon l'invention comprend une étape de mesure d'une pluralité de caractéristiques de l'eau, et la détermination du paramètre caractéristique de l'eau se fait en fonction d'au moins une parmi la pluralité de caractéristiques mesurées de l'eau.

Selon un mode de réalisation, le procédé de contrôle selon l'invention comprend en outre une étape de mesure de la concentration de bactéries dans l'eau par un second capteur positionné à une seconde localisation dans le réseau de distribution d'eau, et la détermination du paramètre caractéristique de l'eau se fait en fonction de la concentration de bactéries dans l'eau mesurée par le second capteur.

Avantageusement, l'action correctrice comprend une étape d'injection d'un produit apte à contrer le développement des bactéries.

Avantageusement, le procédé de contrôle selon l'invention comprend en outre une étape d'asservissement de l'action correctrice en fonction d'un écart entre la concentration de bactéries dans l'eau mesurée par le premier capteur et la valeur instantanée variable.

L'invention concerne aussi un dispositif (10, 100) de contrôle de la concentration de bactéries dans l'eau d'un réseau de distribution (11) d'eau, caractérisé en ce qu'il comprend :
- un premier capteur (12) positionné à une première localisation (13) dans le réseau de distribution (11) d'eau, destiné à mesurer une concentration de bactéries (14) dans l'eau à la première localisation (13),
- un calculateur (15) destiné à déterminer une unique valeur instantanée variable (16) de la concentration de bactéries attendue dans l'eau à la première localisation (13), valeur évolutive et régulièrement mise à jour, en fonction d'un paramètre comprenant des caractéristiques physico-chimiques et/ou bactériologiques de l'eau,
- un comparateur (17) destiné à comparer la concentration de bactéries (14) dans l'eau mesurée par le premier capteur (12) avec la valeur instantanée variable (16) à une fréquence prédéfinie.

Selon un mode de réalisation de l'invention, le dispositif de contrôle comprend une unité de correction destinée à agir sur la concentration de bactéries dans l'eau si la concentration de bactéries dans l'eau mesurée par le premier capteur est supérieure à la valeur instantanée variable.

Selon un mode de réalisation de l'invention, le dispositif de contrôle comprend une base de données destinée à sauvegarder la concentration de bactéries dans l'eau mesurée par le premier capteur de façon à constituer un historique des concentrations de bactéries dans l'eau mesurées par le premier capteur, et le calculateur est configuré pour déterminer le paramètre caractéristique de l'eau en fonction de l'historique des concentrations de bactéries dans l'eau mesurées par le premier capteur.

Selon un autre mode de réalisation de l'invention, le dispositif de contrôle comprend un dispositif de mesure d'une pluralité de caractéristiques de l'eau, et le calculateur est configuré pour déterminer le paramètre caractéristique de l'eau en fonction d'au moins une parmi la pluralité de caractéristiques mesurées de l'eau.

Selon un autre mode de réalisation de l'invention, le dispositif de contrôle comprend un second capteur positionné à une seconde localisation dans le réseau de distribution d'eau, destiné à mesurer une concentration de bactéries dans l'eau à la seconde localisation, et le calculateur est configuré pour déterminer le paramètre caractéristique de l'eau en fonction de la concentration de bactéries dans l'eau mesurée par le second capteur.

Avantageusement, l'unité de correction comprend un dispositif d'injection d'un produit apte à contrer le développement des bactéries.

Avantageusement, le dispositif de contrôle comprend un asservissement de l'unité de correction en fonction d'un écart entre la concentration de bactéries dans l'eau mesurée par le premier capteur et la valeur instantanée variable.

L'invention sera mieux comprise et d'autres avantages apparaîtront à la lecture de la description détaillée d'un mode de réalisation donné à titre d'exemple, description illustrée par le dessin joint dans lequel :
- la figure 1 représente schématiquement les étapes d'un procédé de contrôle de la concentration de bactéries dans l'eau d'un réseau de distribution d'eau selon l'invention,
- la figure 2 représente schématiquement un mode de réalisation d'un dispositif de contrôle de la concentration de bactéries dans l'eau d'un réseau de distribution d'eau selon l'invention,
- la figure 3 représente schématiquement un autre mode de réalisation d'un dispositif de contrôle de la concentration de bactéries dans l'eau d'un réseau de distribution d'eau selon l'invention.

Par souci de clarté, les mêmes éléments porteront les mêmes repères dans les différentes figures.

Dans la description, l'invention est décrite avec l'exemple d'eaux résiduaires. Cependant, l'invention est applicable à tout autre liquide contenant des particules.

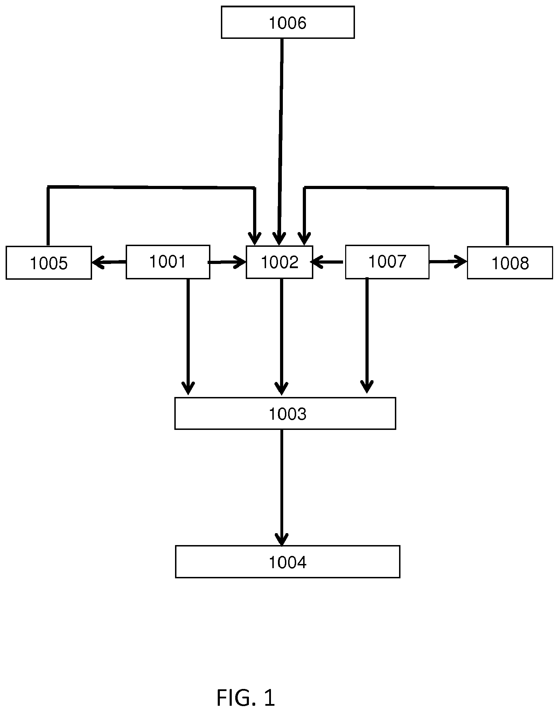

La **figure 1** représente schématiquement les étapes d'un procédé de contrôle de la concentration de bactéries dans l'eau d'un réseau de distribution d'eau selon l'invention. Le procédé de contrôle de la concentration de bactéries dans l'eau d'un réseau de distribution d'eau comprend une étape 1001 de mesure de la concentration de bactéries dans l'eau par un premier capteur positionné à une première localisation dans le réseau de distribution d'eau. Le procédé de contrôle selon l'invention comprend une étape 1002 de détermination d'une valeur instantanée variable de la concentration de bactéries attendue dans l'eau à la première localisation en fonction d'un paramètre caractéristique de l'eau. Comme expliqué précédemment, il est possible de préétablir une valeur de seuil que la concentration de bactéries dans l'eau ne devrait pas dépasser de façon empirique. Mais il a été montré que cette valeur ne prend pas forcément en compte l'évolution du système dans sa globalité. En effet, il est tout à fait possible qu'à un instant donné, la concentration de bactéries mesurée dans l'eau dépasse une valeur de seuil empirique du fait par exemple de l'évolution normale des conditions extérieures. Le fait de déterminer une valeur instantanée variable en fonction d'un paramètre caractéristique de l'eau permet de tenir compte des changements normaux ou anormaux du réseau de distribution d'eau. Le paramètre caractéristique de l'eau est un paramètre qui évolue et qui est pris en compte par une mesure en ligne de la concentration des bactéries ainsi que d'autres paramètres comme explicité plus loin dans la description. Autrement dit, la valeur instantanée variable n'est pas une valeur figée. Cette valeur est évolutive au cours du déroulement du procédé selon l'invention. En d'autres termes, la valeur instantanée variable de la concentration de bactéries attendue correspond à un seuil qui prend en compte les conditions environnantes du réseau de distribution d'eau. Les différentes qualités d'eau correspondant à différentes sources, différents procédés de traitement et différents régimes hydrauliques peuvent ainsi être prises en compte sans causer de fausses alarmes. Les évolutions de niveau de concentration en bactéries dues à des phénomènes ayant un impact sur les autres paramètres mais ne nécessitant pas d'intervention peuvent ainsi être discriminées via un seuil reflétant le niveau attendu. Ce seuil est la valeur instantanée variable. Cette valeur instantanée variable est donc parfaitement adaptée au contrôle de la concentration de bactéries dans l'eau.

Ensuite a lieu une étape 1003 de comparaison de la concentration de bactéries dans l'eau mesurée par le premier capteur avec la valeur instantanée variable. Notons également que la comparaison peut être effectuée entre la concentration de bactéries dans l'eau mesurée par le premier capteur avec la valeur instantanée variable ou bien avec la valeur instantanée variable à laquelle on ajoute un écart, pour intégrer une certaine tolérance dans ce qui constitue notre seuil.

On peut noter que l'étape 1002 de détermination de la valeur instantanée variable de la concentration de bactéries attendue à la première localisation peut avoir lieu avant, pendant ou juste après l'étape 1001 de mesure. En tout état de cause, l'étape 1002 a nécessairement lieu avant l'étape 1003 de comparaison de la concentration de bactéries dans l'eau mesurée par le premier capteur avec la valeur instantanée variable.

La valeur instantanée variable dépend des conditions environnantes et une unique valeur instantanée variable est déterminée avant l'étape 1003 de comparaison, c'est-à-dire qu'avant chaque comparaison, une nouvelle valeur instantanée variable est déterminée. Autrement dit, comme la valeur instantanée variable est évolutive et régulièrement mise à jour, il n'y a pas besoin de sauvegarder cette valeur dans une base de données universelle. Un simple stockage de l'historique suffit. Le procédé de contrôle selon l'invention est donc plus facile à gérer.

Le paramètre caractéristique comprend les conditions ennvironnantes du réseau d'eau et l'historique des mesures effectuées. Ainsi, le paramètre caractéristique est un vecteur de valeurs issues de ces deux composantes : état actuel des caractéristiques de l'eau et l'historique des mesures.

Il est par ailleurs important de souligner que l'étape 1001 de mesure de la concentration de bactéries dans l'eau par le premier capteur positionné à la première localisation dans le réseau de distribution d'eau se fait sur site et non en laboratoire. L'invention permet donc de faire un suivi en continu de la microflore de l'eau et d'effectuer des mesures en temps réel.

Enfin, le procédé de contrôle selon l'invention peut comprendre une étape 1004 d'action correctrice agissant sur la concentration de bactéries dans l'eau si la concentration de bactéries dans l'eau mesurée par le premier capteur est supérieure à la valeur instantanée variable. Quand le procédé de contrôle comprend l'étape 1004 d'action correctrice, l'action correctrice a généralement lieu si la concentration de bactéries dans l'eau mesurée par le premier capteur est significativement supérieure à la valeur instantanée variable. Il est ici question de la concentration de bactéries mesurée comparée à la valeur instantanée variable. Le caractère significatif de ce dépassement traduit la prise en compte d'un nombre de pas de temps supérieur à 1 avec un dépassement dans une fenêtre temporelle déterminée. Ceci permet d'éviter de déclencher l'action correctrice sur la base d'un unique dépassement pouvant correspondre à une valeur aberrante, et ainsi de limiter les fausses alarmes. L'invention s'applique aussi, sur le même principe, à l'augmentation de la concentration de bactéries dans l'eau mesurée par le premier capteur qui serait alors comparée à une valeur instantanée variable d'augmentation en fonction d'un paramètre caractéristique de l'eau tenant compte de l'évolution de la concentration de bactéries.

Selon l'invention, l'étape 1003 de comparaison se fait à une fréquence prédéfinie, par exemple toutes les 10 minutes. Selon des variantes de réalisation non couvertes par la présente invention, l'étape 1003 de comparaison se fait sur un intervalle de temps et/ou en prenant en compte une série de mesures de concentration de bactéries.

Selon un mode de réalisation de l'invention, le procédé de contrôle peut comprendre une étape 1005 de sauvegarde de la concentration de bactéries dans l'eau mesurée à l'étape 1001 par le premier capteur dans une base de données de façon à constituer un historique des concentrations debactéries dans l'eau mesurées par le premier capteur. La détermination du paramètre caractéristique de l'eau permettant de déterminer la valeur instantanée variable peut alors se faire en fonction de l'historique des concentrations de bactéries dans l'eau mesurées par le premier capteur.

Selon un mode de réalisation, le paramètre caractéristique est représenté par les paramètres estimés d'une distribution statistique de valeurs observées de concentrations en bactéries, comprenant par exemple une moyenne et un écart-type empiriques. Selon un autre mode de réalisation, le paramètre caractéristique est représenté par une classe discrète représentant une qualité d'eau observée pour la mesure bactériologique.

Selon un autre mode de réalisation de l'invention, le procédé de contrôle peut comprendre une étape 1006 de mesure d'une pluralité de caractéristiques de l'eau, et la détermination du paramètre caractéristique de l'eau se fait en fonction d'au moins une parmi la pluralité de caractéristiques mesurées de l'eau. Selon un mode de réalisation, le paramètre caractéristique peut donc être exprimé par les paramètres estimés des distributions statistiques de valeurs observées des différentes caractéristiques physico-chimiques et bactériologiques mesurées. Selon un autre mode de réalisation, le paramètre caractéristique peut être représenté par une classe discrète représentant une qualité d'eau observée pour la mesure bactériologique.

Parmi les caractéristiques de l'eau, on peut citer, par exemple et de manière non exhaustive, la pression, le débit, le pH de l'eau, sa température, sa conductivité, sa teneur en chlore, sa turbidité, sa teneur en carbone organique totale ou la concentration d'oxygène dissous dans l'eau. La mesure de ces caractéristiques peut être réalisée en ligne ou bien en laboratoire. La valeur considérée pour chacune des caractéristiques peut être un minimum de la valeur, un maximum de la valeur, une moyenne ou un quantile. Le paramètre caractéristique de l'eau peut être déterminé en fonction d'une seule caractéristique de l'eau, par exemple la température ou la teneur en chlore, ou bien de plusieurs caractéristiques par exemple la température de l'eau, sa teneur en chlore et son pH. Et une ou plusieurs de ces caractéristiques de l'eau peut être un indicateur sur une évolution de la concentration de bactéries dans l'eau. C'est pour cette raison qu'il est important qu'il(s) soi(en)t pris en compte dans la détermination du paramètre caractéristique de l'eau afin de déterminer une valeur instantanée, qui est donc variable puisqu'elle peut changer de valeur en fonction de changement de valeur du ou des caractéristique(s) de l'eau, à partir de laquelle une alarme peut être déclenchée si la concentration de bactéries mesurée dépasse cette valeur instantanée ainsi déterminée.

On peut noter que la détermination de la valeur instantanée variable peut être basée sur les autres paramètres mais pas uniquement, il est nécessaire d'avoir au moins quelques valeurs de concentrations de bactéries pour déterminer le ou les niveaux de base.

Selon un autre mode de réalisation de l'invention, le procédé de contrôle peut comprendre en outre une étape 1007 de mesure de la concentration de bactéries dans l'eau par un second capteur positionné à une seconde localisation dans le réseau de distribution d'eau, et la détermination du paramètre caractéristique de l'eau se fait en fonction de la concentration de bactéries dans l'eau mesurée par le second capteur. L'étape 1007 présente deux avantages majeurs. Dans le cas où le second capteur est positionné à proximité du premier capteur dans une même conduite du réseau de distribution d'eau, sans ramification de conduites entre les deux capteurs, les concentrations de bactéries mesurées par les deux capteurs doivent être sensiblement les mêmes. Si les concentrations mesurées par les deux capteurs diffèrent trop, cela peut être le signe qu'au moins un des deux capteurs est défectueux. Dans ce cas, l'étape 1007 permet la vérification du bon état des capteurs de concentration de bactéries. Dans le cas où le second capteur est positionné à distance du premier capteur, que ce soit dans une même conduite ou éloigné dans le réseau de distribution d'eau, voire dans une autre conduite ramifiée par rapport à la conduite du premier capteur, le second capteur donne une concentration de bactéries mesurée à une seconde localisation. Cette information de concentration à un autre endroit du réseau peut être utilisée pour le calcul du paramètre caractéristique de l'eau afin d'améliorer la précision de la valeur de la concentration de bactéries attendue à la première localisation pour un état du réseau de distribution donné à cet instant.

Sur le même principe et de façon analogue, le procédé de contrôle peut comprendre en outre une étape de mesure de la concentration de bactéries dans l'eau par un troisième capteur positionné à une troisième localisation dans le réseau de distribution d'eau. Le même raisonnement s'applique avec plusieurs autres capteurs.

Par ailleurs, le procédé de contrôle peut comprendre une étape 1008 de sauvegarde de la concentration de bactéries dans l'eau mesurée à l'étape 1007 par le second capteur dans une base de données de façon à constituer un historique des concentrations de bactéries dans l'eau mesurées par le second capteur. La détermination du paramètre caractéristique de l'eau permettant de déterminer la valeur instantanée variable peut alors se faire en fonction de l'historique des concentrations de bactéries dans l'eau mesurées par le second capteur aussi.

De manière plus précise, la détermination du paramètre caractéristique de l'eau permettant de déterminer la valeur instantanée variable peut alors se faire en fonction à la fois de l'historique des concentrations de bactéries dans l'eau mesurées par le premier et le second capteurs et aussi des concentrations de bactéries dans l'eau mesurées à la première et seconde localisations à cet instant.

Avantageusement, la détermination du paramètre caractéristique de l'eau permettant de déterminer la valeur instantanée variable peut alors se faire en fonction de l'historique des concentrations de bactéries dans l'eau mesurées par le premier et le second capteurs, en fonction des concentrations de bactéries dans l'eau mesurées à la première et seconde localisations à cet instant et aussi en fonction d'au moins une parmi la pluralité de caractéristiques mesurées de l'eau. Il en résulte que la valeur instantanée variable, qui est la valeur à laquelle la concentration de bactéries mesurée par le premier capteur est comparée et qui est déterminante pour lancer le signal d'alarme si la concentration mesurée est trop élevée par rapport à ce qu'elle est sensée être, s'adapte aux différentes données disponibles sur le réseau de distribution, et permet de prendre en compte les caractéristiques propres à chaque eau.

On peut noter que l'invention s'applique de façon analogue à plusieurs autres capteurs dans la même conduite d'eau et/ou à d'autres localisations dans le réseau de distribution d'eau, et de même pour la sauvegarde des concentrations mesurées pour constituer l'historique des concentrations mesurées résultantes. Il est ainsi possible de déterminer la valeur instantanée variable de la concentration de bactéries attendue à une localisation en fonction, entre autres, de la concentration de bactéries mesurée à plusieurs autres localisations dans le réseau. Il en résulte une valeur instantanée variable correspondant tout à fait à l'état du réseau de distribution dans son ensemble.

Avantageusement, l'action correctrice de l'étape 1004 comprend une étape d'injection d'un produit apte à contrer le développement des bactéries. L'action correctrice prend généralement la forme d'injection d'une solution désinfectante, par exemple du chlore ou autre biocide dans le réseau de distribution. Selon un autre mode de réalisation, l'action correctrice de l'étape 1004 comprend une purge de la partie du réseau concernée par le dépassement de la valeur instantanée variable.

Avantageusement, le procédé de contrôle selon l'invention comprend en outre une étape d'asservissement de l'action correctrice en fonction d'un écart entre la concentration de bactéries dans l'eau mesurée par le premier capteur et la valeur instantanée variable. L'écart donne une indication sur la différence entre la concentration mesurée et la concentration attendue à la première localisation. Selon cette différence, la quantité de produit à injecter est plus ou moins importante. L'étape d'asservissement de l'action correctrice permet d'adapter la quantité de produit injecté, par exemple de chlore, dans le réseau. Une fois le chlore injecté, une autre mesure de la concentration de bactéries est faite par le premier capteur, un autre écart est calculé et selon l'écart obtenu, la quantité de chlore à injecter est adaptée. Si la concentration de bactéries mesurée est encore trop élevée par rapport à ce qu'elle est sensée être, la quantité de chlore injectée peut être augmentée ou maintenue constante jusqu'à ce que le premier capteur mette en valeur une baisse de la concentration de bactéries mesurée à la première localisation. Si la concentration de bactéries mesurée est inférieure à la valeur instantanée variable, l'injection de chlore est stoppée.

Le procédé de contrôle selon l'invention rend ainsi possible un contrôle de la concentration de bactéries dans un réseau d'eau de façon évolutive et adaptative en fonction de l'état du réseau dans sa globalité et qui prend en compte les variations de paramètres annexes tels que les caractéristiques de l'eau et/ou l'historique de concentrations à un ou plusieurs autres endroits dans le réseau de distribution d'eau.

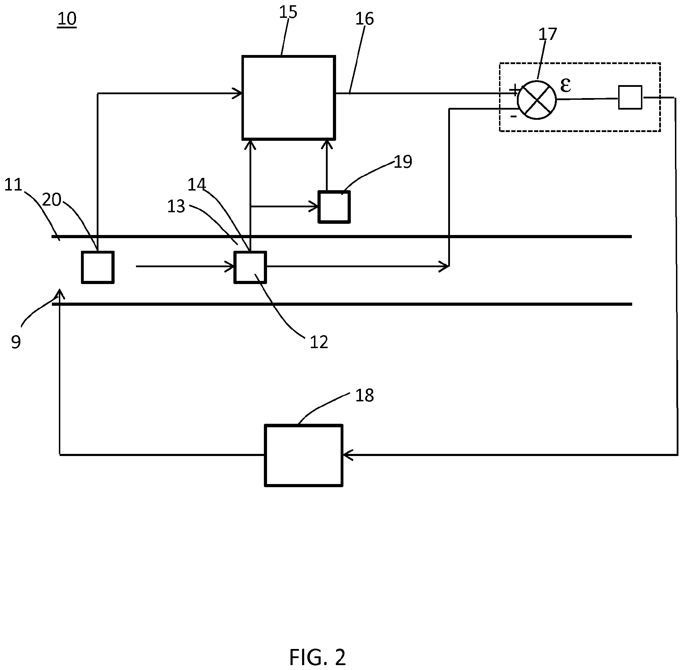

La **figure 2** représente schématiquement un mode de réalisation d'un dispositif 10 de contrôle de la concentration de bactéries dans l'eau d'un réseau de distribution d'eau selon l'invention. Le dispositif 10 de contrôle de la concentration de bactéries dans l'eau d'un réseau 11 de distribution d'eau peut être utilisé pour mettre en oeuvre le procédé tel que décrit à la figure 1. Le dispositif 10 comprend un premier capteur 12 positionné à une première localisation 13 dans le réseau 11 de distribution d'eau, destiné à mesurer une concentration de bactéries 14 dans l'eau à la première localisation 13. Le dispositif 10 comprend un calculateur 15 destiné à déterminer une valeur instantanée variable 16 de la concentration de bactéries attendue dans l'eau à la première localisation 13 en fonction d'un paramètre caractéristique de l'eau. Le dispositif 10 comprend un comparateur 17 destiné à comparer la concentration de bactéries 14 dans l'eau mesurée par le premier capteur 12 avec la valeur instantanée variable 16. Le dispositif 10 comprend une unité de correction 18 destinée à agir sur la concentration de bactéries dans l'eau si la concentration de bactéries 14 dans l'eau mesurée par le premier capteur 13 est supérieure à la valeur instantanée variable 16.

Selon un mode de réalisation de l'invention, le dispositif 10 peut comprendre une base de données 19 destinée à sauvegarder la concentration de bactéries 14 dans l'eau mesurée par le premier capteur 12 de façon à constituer un historique des concentrations de bactéries dans l'eau mesurées par le premier capteur 12. La base de données 19 peut être intégrée au calculateur 15 ou au capteur 12 ou sur un support extérieur au calculateur 15 et au capteur 12. Le calculateur 15 est alors configuré pour déterminer le paramètre caractéristique de l'eau en fonction de l'historique des concentrations de bactéries dans l'eau mesurées par le premier capteur 12. Par exemple, le paramètre caractéristique de l'eau peut être calculé par le calculateur 15 en se basant sur l'historique des concentrations de bactéries dans l'eau mesurées par le premier capteur 12. Le paramètre caractéristique de l'eau permet de déterminer la valeur instantanée variable 16. Grâce à la base de données 19, il est possible d'effectuer une série temporelle de mesures de la concentration de bactéries 14, sur un jour, une semaine, un mois ou plus, à intervalles réguliers ou irréguliers. Ces mesures sont sauvegardées dans la base de données 19. Et il est alors possible d'établir, en fonctionnement normal du réseau de distribution d'eau, une valeur moyenne de la concentration de bactéries à la première localisation et une amplitude des fluctuations normales autour de la valeur moyenne, ou une autre définition d'un intervalle de fluctuation normale. On peut ainsi étudier la distribution statistique observée dans l'historique afin de mieux déterminer la valeur instantanée variable qui correspond à une valeur attendue pour la concentration de bactéries dans l'eau à la première localisation. Un exemple est de considérer une valeur instantanée variable décrite selon une distribution gaussienne et le paramètre caractéristique de l'eau comme étant un certain nombre d'écart type, ou une autre distribution de probabilité paramétrique. Un autre exemple permet de prendre en compte la saisonnalité observée de la concentration en bactéries en séparant la tendance saisonnière du signal initial, afin de déterminer une valeur instantanée reflétant également cette saisonnalité. Un autre exemple utilise les techniques de séries chronologiques de décomposition du signal en différentes composantes ayant leur périodicité propre. Un autre exemple consiste à obtenir par des méthodes non-paramétriques ou d'apprentissage statistique une expression de la valeur instantanée variable en fonction du paramètre caractéristique de l'eau.

Le dispositif 10 de contrôle selon l'invention peut comprendre un dispositif 20 de mesure d'une pluralité de caractéristiques de l'eau. Comme expliqué précédemment, parmi les caractéristiques de l'eau, on peut citer, par exemple et de manière non exhaustive, la pression, le débit de l'eau, sa température, son pH, sa conductivité, sa teneur en chlore, sa turbidité ou la concentration d'oxygène dissous dans l'eau. La mesure de ces caractéristiques peut être réalisée en ligne par le dispositif 20 ou bien en laboratoire. La valeur considérée pour chacune des caractéristiques peut être un minimum de la valeur, un maximum de la valeur, une moyenne ou un quantile. Le calculateur 15 est configuré pour déterminer le paramètre caractéristique de l'eau en fonction d'au moins une parmi la pluralité de caractéristiques mesurées de l'eau. Dans ce cas, le paramètre caractéristique de l'eau permet de prendre en compte l'évolution de la concentration de bactéries mesurée 14 due à un fonctionnement normal du réseau. Autrement dit, si la température de l'eau augmente, il est fort probable que la concentration de bactéries mesurée 14 augmente elle aussi. Cette augmentation de la concentration de bactéries mesurée 14 n'est donc pas la conséquence d'un dysfonctionnement du réseau de distribution et ne nécessite pas forcément la même action correctrice que lors d'un autre évènement impactant la concentration de bactéries. Il est donc important de tenir compte de cette différenciation et de pouvoir faire évoluer la valeur instantanée variable 16 en fonction des conditions environnantes du réseau. La détermination de la valeur instantanée variable de la concentration en bactéries peut ainsi être réalisée avec un modèle prédictif sur la base des différents paramètres caractéristiques. En particulier différents modèles prédictifs sont testés dans un processus de validation croisée sur les données passées disponibles, notamment grâce à la base de données obtenue grâce à la sauvegarde de la concentration de bactéries mesurée à la première localisation. Le modèle le plus pertinent, c'est-à-dire permettant les performances les plus avantageuses (précision, sensibilité aux valeurs aberrantes, faible temps de calcul ...), est alors sélectionné pour le calcul de la valeur instantanée variable. Parmi les modèles prédictifs testés se trouvent notamment les algorithmes d'apprentissage statistique, en particulier les arbres de décision et forêts aléatoires, les modèles linéaires généralisés et les réseaux de neurones.

L'unité de correction 18 du dispositif 10 de contrôle comprend un dispositif d'injection 9 d'un produit apte à contrer le développement des bactéries. L'unité de correction 18 consiste généralement en un injecteur d'une solution désinfectante, par exemple du chlore ou autre biocide dans le réseau de distribution. Avantageusement, l'injecteur est positionné bien en amont du premier capteur 12 de façon à ce que les mesures effectuées par le premier capteur 12 ultérieurement à l'action correctrice puissent prendre en compte les résultats de l'action correctrice.

Avantageusement, le dispositif 10 de contrôle comprend un asservissement de l'unité de correction 18 en fonction d'un écart entre la concentration de bactéries dans l'eau 14 mesurée par le premier capteur 12 et la valeur instantanée variable 16. Selon l'écart entre la concentration mesurée et la concentration attendue à la première localisation 13, l'unité de correction 18 peut adapter la quantité de produit à injecter. L'asservissement de l'unité de correction 18 permet d'adapter la quantité de produit injecté, par exemple de chlore, dans le réseau. Une fois le chlore injecté, une autre mesure de la concentration de bactéries 14 est faite par le premier capteur 12, un autre écart est calculé et selon l'écart obtenu, la quantité de chlore à injecter est adaptée. Si la concentration de bactéries mesurée est encore trop élevée par rapport à ce qu'elle est sensée être, la quantité de chlore injectée peut être augmentée ou maintenue constante jusqu'à ce que le premier capteur 12 mette en valeur une baisse de la concentration de bactéries mesurée à la première localisation 13. Si la concentration de bactéries mesurée 14 est inférieure à la valeur instantanée variable 16, l'injection de chlore est stoppée. Un tel asservissement permet une régulation fiable et précise de la concentration de bactéries dans le réseau de distribution.

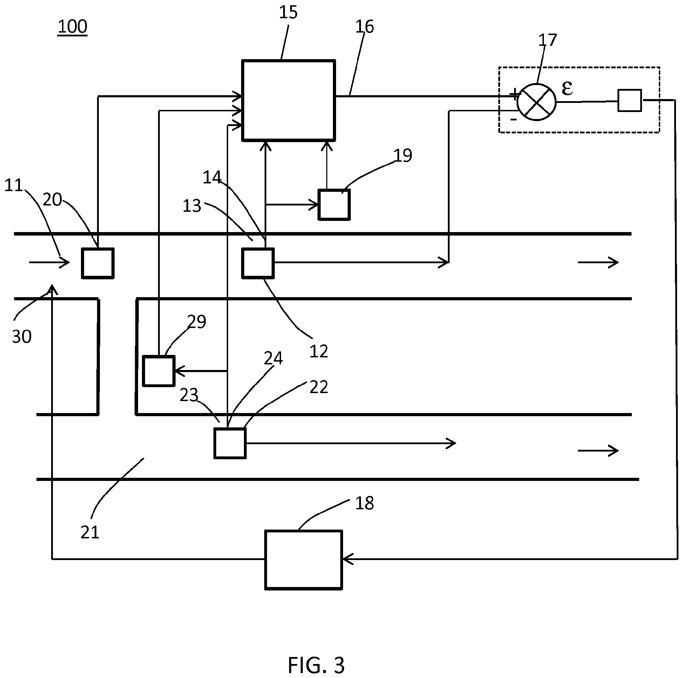

La **figure 3** représente schématiquement un autre mode de réalisation d'un dispositif 100 de contrôle de la concentration de bactéries dans l'eau d'un réseau de distribution d'eau selon l'invention. Le dispositif 100 de contrôle de la concentration de bactéries dans l'eau d'un réseau 11 de distribution d'eau peut être utilisé pour mettre en oeuvre le procédé tel que décrit à la figure 1. Tous les éléments du dispositif 100 de la figure 3 sont identiques aux éléments du dispositif 10 de la figure 2. Dans le mode de réalisation de la figure 3, le dispositif 100 est présenté dans le cas d'un réseau de distribution d'eau avec deux conduites 11 et 21 parallèles et ramifiées entre elles. Sur les figures 2 et 3, l'eau circule de la gauche vers la droite. Sur la figure 3, la ramification entre les deux conduites 11 et 21 se trouve en amont du premier capteur 12.

Dans ce mode de réalisation, le dispositif 100 de contrôle comprend un second capteur 22 positionné à une seconde localisation 23 dans le réseau de distribution d'eau, destiné à mesurer une concentration de bactéries 24 dans l'eau à la seconde localisation 23. Le calculateur 15 est configuré pour déterminer le paramètre caractéristique de l'eau en fonction de la concentration de bactéries dans l'eau 24 mesurée par le second capteur 22. Le second capteur 22 donne une concentration de bactéries mesurée 24 à une seconde localisation 23. Cette information de concentration à un autre endroit du réseau peut être utilisée pour le calcul du paramètre caractéristique de l'eau afin d'améliorer la précision de la valeur de la concentration de bactéries attendue à la première localisation 13 pour un état du réseau de distribution donné à cet instant.

Pour faciliter la lecture et la compréhension, un dispositif 100 avec un premier et un second capteur est décrit. Il est bien évident que l'invention concerne aussi, de manière analogue, un dispositif de contrôle avec plus que deux capteurs.

L'amélioration de l'estimation du paramètre caractéristique de l'eau au point du premier capteur peut être obtenue à partir des données historiques et d'une analyse de la corrélation linéaire ou non-linéaire entre les paramètres aux deux localisations. La détermination de la valeur instantanée variable de la concentration en bactéries peut ainsi être réalisée avec un modèle prédictif sur la base des différents paramètres caractéristiques. En particulier différents modèles prédictifs sont testés dans un processus de validation croisée sur les données passées disponibles, notamment grâce à la base de données obtenue grâce à la sauvegarde de la concentration de bactéries mesurée à la première localisation. Le modèle le plus pertinent, c'est-à-dire permettant les performances les plus avantageuses (précision, sensibilité aux valeurs aberrantes, faible temps de calcul ...), est alors sélectionné pour le calcul de la valeur instantanée variable. Parmi les modèles prédictifs testés se trouvent notamment les algorithmes d'apprentissage statistique, en particulier les arbres de décision et forêts aléatoires, les modèles linéaires généralisés et les réseaux de neurones.

Avantageusement, la détermination du paramètre caractéristique de l'eau permettant de déterminer la valeur instantanée variable 16 peut alors se faire en fonction de l'historique des concentrations de bactéries dans l'eau mesurées 14, 24 par le premier et le second capteurs 12, 22, en fonction des concentrations de bactéries dans l'eau mesurées 14, 24 à la première et seconde localisations 13, 23 à cet instant et aussi en fonction d'au moins une parmi la pluralité de caractéristiques mesurées de l'eau. Il en résulte que la valeur instantanée variable 16, qui est la valeur à laquelle la concentration de bactéries mesurée 14 par le premier capteur 12 est comparée et qui est déterminante pour lancer le signal d'alarme si la concentration mesurée 14 est trop élevée par rapport à ce qu'elle est sensée être, s'adapte aux différentes données disponibles sur le réseau de distribution, et permet de prendre en compte les caractéristiques propres à chaque eau.

Comme déjà expliqué, on peut noter que l'invention s'applique de façon analogue à un dispositif avec plusieurs autres capteurs dans la même conduite d'eau et/ou à d'autres localisations dans le réseau de distribution d'eau, et de même pour la sauvegarde des concentrations mesurées pour constituer l'historique des concentrations mesurées résultantes. Il est ainsi possible de déterminer la valeur instantanée variable 16 de la concentration de bactéries attendue à une localisation en fonction, entre autres, de la concentration de bactéries mesurée à plusieurs autres localisations dans le réseau. Il en résulte une valeur instantanée variable 16 correspondant tout à fait à l'état du réseau de distribution dans son ensemble.

L'invention traite ici d'un calculateur 15 déterminant une valeur instantanée variable 16 pour une concentration de bactéries attendue à une localisation précise, c'est-à-dire la première localisation 13. Il est bien évident que l'invention s'applique de manière analogue pour une autre, ou plusieurs autres, valeur(s) instantanée(s) variable(s) pour une autre, ou plusieurs autres, localisation(s). Le dispositif de contrôle peut alors utiliser un seul calculateur 15 configuré pour déterminer plusieurs valeurs instantanées variables ou bien plusieurs calculateurs peuvent être mis en oeuvre à cet effet. De même, le dispositif selon l'invention peut comprendre une seule ou plusieurs unités de correction pour agir sur les concentrations de bactéries à différentes localisations dans le réseau de distribution. Enfin, il est tout à fait envisageable dans le cadre de l'invention de prévoir un asservissement des unités de correction permettant d'agir en fonction des écarts entre les concentrations de bactéries mesurées et les valeurs instantanées variables correspondantes.

En outre, l'étape 1002 du procédé de contrôle selon l'invention peut comprendre une phase d'apprentissage permettant au calculateur 15 de caractériser les situations normales des situations anormales, en se basant notamment sur l'historique des concentrations mesurées et toute autre information disponible. La phase d'apprentissage peut être répétée périodiquement afin de mettre à jour le modèle de traitement des données. De ce fait, le calculateur 15 peut être configuré pour réaliser une phase d'apprentissage de sorte à pouvoir caractériser les situations normales des situations anormales, en se basant sur l'historique des concentrations mesurées et toute autre information disponible.

Ainsi, le procédé selon l'invention et le dispositif selon l'invention permettent d'améliorer l'utilisation opérationnelle des données issues du suivi de la bactériologie pour les réseaux de distribution d'eau. Le traitement des données permet ainsi à un opérateur de réseau une meilleure visualisation du comportement du réseau et le déclenchement des actions correctives et préventives avec une plus grande efficacité.

L'invention peut être appliquée au suivi de tout type d'eau, par exemple les eaux de réseau de refroidissement, les eaux naturelles, environnementales, de process ou bien à la réutilisation d'eaux usées. Le premier capteur 12 a été décrit comme étant positionné à la première localisation 13 mais il peut être en dérivation avec le milieu où l'eau à contrôler est en circulation. Les mesures de concentration sont explicitées dans cette demande pour des bactéries, mais l'invention s'applique de façon similaire à un groupe de bactéries, genre ou espèce bactérienne, aussi indicateurs de la qualité microbiologique de l'eau.

## Revendications

1. Procédé de contrôle de la concentration de bactéries dans l'eau d'un réseau de distribution d'eau dans des conditions environnantes, **caractérisé en ce qu'**il comprend les étapes suivantes:
• mesure de la concentration de bactéries dans l'eau par un premier capteur positionné à une première localisation dans le réseau de distribution d'eau (étape 1001),
• détermination d'une unique valeur instantanée variable de la concentration de bactéries attendue dans l'eau à la première localisation, valeur évolutive et régulièrement mise à jour, en fonction d'un paramètre comprenant des caractéristiques physico-chimiques et/ou bactériologiques de l'eau, ledit paramètre étant évolutif et prenant en compte les conditions environnantes du réseau d'eau (étape 1002),
• à une fréquence prédéfinie : comparaison de la concentration de bactéries dans l'eau mesurée par le premier capteur avec la valeur instantanée variable (étape 1003).

2. Procédé de contrôle selon la revendication 1, **caractérisé en ce qu'**il comprend en outre une étape (1004) d'action correctrice agissant sur la concentration de bactéries dans l'eau si la concentration de bactéries dans l'eau mesurée par le premier capteur est supérieure à la valeur instantanée variable.

3. Procédé de contrôle selon l'une quelconque des revendications 1 ou 2, **caractérisé en ce qu'**il comprend une étape (1005) de sauvegarde de la concentration de bactéries dans l'eau mesurée par le premier capteur dans une base de données de façon à constituer un historique des concentrations de bactéries dans l'eau mesurées par le premier capteur, et **en ce que** la détermination du paramètre caractéristique de l'eau se fait en fonction de l'historique des concentrations de bactéries dans l'eau mesurées par le premier capteur.

4. Procédé de contrôle selon l'une quelconque des revendications précédentes, **caractérisé en ce qu'**il comprend une étape (1006) de mesure d'une pluralité de caractéristiques de l'eau, et **en ce que** la détermination du paramètre caractéristique de l'eau se fait en fonction d'au moins une parmi la pluralité de caractéristiques mesurées de l'eau.

5. Procédé de contrôle selon l'une quelconque des revendications précédentes, **caractérisé en ce qu'**il comprend en outre une étape (1007) de mesure de la concentration de bactéries dans l'eau par un second capteur positionné à une seconde localisation dans le réseau de distribution d'eau, et **en ce que** la détermination du paramètre caractéristique de l'eau se fait en fonction de la concentration de bactéries dans l'eau mesurée par le second capteur.

6. Procédé de contrôle selon l'une quelconque des revendications 2 à 5, **caractérisé en ce que** l'action correctrice (étape 1004) comprend une étape d'injection d'un produit apte à contrer le développement des bactéries.

7. Procédé de contrôle selon l'une quelconque des revendications 2 à 6, **caractérisé en ce qu'**il comprend en outre une étape d'asservissement de l'action correctrice en fonction d'un écart entre la concentration de bactéries dans l'eau mesurée par le premier capteur et la valeur instantanée variable.

8. Dispositif (10, 100) de contrôle de la concentration de bactéries dans l'eau d'un réseau de distribution (11) d'eau, **caractérisé en ce qu'**il comprend :
• un premier capteur (12) positionné à une première localisation (13) dans le réseau de distribution (11) d'eau, destiné à mesurer une concentration de bactéries (14) dans l'eau à la première localisation (13),
• un calculateur (15) destiné à déterminer une unique valeur instantanée variable (16) de la concentration de bactéries attendue dans l'eau à la première localisation (13), valeur évolutive et régulièrement mise à jour, en fonction d'un paramètre comprenant des caractéristiques physico-chimiques et/ou bactériologiques de l'eau,
• un comparateur (17) destiné à comparer la concentration de bactéries (14) dans l'eau mesurée par le premier capteur (12) avec la valeur instantanée variable (16) à une fréquence prédéfinie.

9. Dispositif (10, 100) selon la revendication 8, **caractérisé en ce qu'**il comprend une unité de correction (18) destinée à agir sur la concentration de bactéries dans l'eau si la concentration de bactéries (14) dans l'eau mesurée par le premier capteur (12) est supérieure à la valeur instantanée variable (16).

10. Dispositif (10, 100) selon l'une quelconque des revendications 8 ou 9, **caractérisé en ce qu'**il comprend une base de données (19) destinée à sauvegarder la concentration de bactéries (14) dans l'eau mesurée par le premier capteur (12) de façon à constituer un historique des concentrations de bactéries (14) dans l'eau mesurées par le premier capteur (12), et **en ce que** le calculateur (15) est configuré pour déterminer le paramètre comprenant des caractéristiques physico-chimiques et/ou bactériologiques de l'eau en fonction de l'historique des concentrations de bactéries dans l'eau (14) mesurées par le premier capteur (12).

11. Dispositif (10, 100) de contrôle selon l'une quelconque des revendications 8 à 10, **caractérisé en ce qu'**il comprend un dispositif (20) de mesure d'une pluralité de caractéristiques de l'eau, et **en ce que** le calculateur (15) est configuré pour déterminer le paramètre caractéristique de l'eau en fonction d'au moins une parmi la pluralité de caractéristiques mesurées de l'eau.

12. Dispositif (100) de contrôle selon l'une quelconque des revendications 8 à 11, **caractérisé en ce qu'**il comprend un second capteur (22) positionné à une seconde localisation (23) dans le réseau de distribution d'eau, destiné à mesurer une concentration de bactéries (24) dans l'eau à la seconde localisation (23), et **en ce que** le calculateur (15) est configuré pour déterminer le paramètre comprenant des caractéristiques physico-chimiques et/ou bactériologiques de l'eau en fonction de la concentration de bactéries dans l'eau (24) mesurée par le second capteur (22).

13. Dispositif (10, 100) de contrôle selon l'une quelconque des revendications 9 à 12, **caractérisé en ce que** l'unité de correction (18) comprend un dispositif d'injection (30) d'un produit apte à contrer le développement des bactéries.

14. Dispositif (10, 100) de contrôle selon l'une quelconque des revendications 9 à 13, **caractérisé en ce qu'**il comprend un asservissement de l'unité de correction (18) en fonction d'un écart entre la concentration de bactéries dans l'eau (14) mesurée par le premier capteur (12) et la valeur instantanée variable (16).

## Patentansprüche

1. Verfahren zur Überwachung der Konzentration von Bakterien im Wasser eines Wasserverteilungsnetzes unter Umgebungsbedingungen, **dadurch gekennzeichnet, dass** es die folgenden Schritte umfasst:
• Messung der Bakterienkonzentration in dem Wasser durch einen ersten Sensor, der an einem ersten Ort in dem Wasserverteilungsnetz positioniert ist (Schritt 1001),
• Bestimmung eines einzigen variablen Momentanwerts der erwarteten Bakterienkonzentration im Wasser an der ersten Stelle, wobei dieser Wert sich entwickelt und regelmäßig aktualisiert wird, in Abhängigkeit von einem Parameter, der physikalisch-chemische und/oder bakteriologische Merkmale des Wassers umfasst, wobei der Parameter sich entwickelt und die Umgebungsbedingungen des Wassernetzes berücksichtigt (Schritt 1002),
• bei einer vorbestimmten Frequenz: Vergleich der vom ersten Sensor gemessenen Bakterienkonzentration im Wasser mit dem variablen Momentanwert (Schritt 1003).

2. Überwachungsverfahren nach Anspruch 1, **dadurch gekennzeichnet, dass** es ferner einen Korrekturmaßnahmenschritt (1004) umfasst, der auf die Bakterienkonzentration im Wasser einwirkt, wenn die vom ersten Sensor gemessene Bakterienkonzentration im Wasser höher als der variable augenblickliche Wert ist.

3. Überwachungsverfahren nach einem der Ansprüche 1 oder 2, **dadurch gekennzeichnet, dass** es einen Schritt (1005) umfasst, bei dem die vom ersten Sensor gemessene Bakterienkonzentration im Wasser in einer Datenbank abgespeichert wird, um eine Historie der vom ersten Sensor gemessenen Bakterienkonzentrationen im Wasser zu bilden, und dass die Bestimmung des charakteristischen Parameters des Wassers in Abhängigkeit von der Historie der vom ersten Sensor gemessenen Bakterienkonzentrationen im Wasser erfolgt.

4. Überwachungsverfahren nach einem der vorhergehenden Ansprüche, **dadurch gekennzeichnet, dass** es einen Schritt (1006) des Messens einer Vielzahl von Merkmalen des Wassers umfasst, und dass die Bestimmung des charakteristischen Parameters des Wassers in Abhängigkeit von mindestens einem aus der Vielzahl von gemessenen Merkmalen des Wassers erfolgt.

5. Überwachungsverfahren nach einem der vorhergehenden Ansprüche, **dadurch gekennzeichnet, dass** es ferner einen Schritt (1007) des Messens der Konzentration von Bakterien im Wasser durch einen zweiten Sensor umfasst, der an einem zweiten Ort im Wasserverteilungsnetz positioniert ist, und dass die Bestimmung des charakteristischen Parameters des Wassers in Abhängigkeit von der Konzentration von Bakterien im Wasser erfolgt, die durch den zweiten Sensor gemessen wurde.

6. Überwachungsverfahren nach einem der Ansprüche 2 bis 5, **dadurch gekennzeichnet, dass** die Korrekturmaßnahme (Schritt 1004) einen Schritt der Injektion eines Produkts umfasst, das in der Lage ist, dem Wachstum von Bakterien entgegenzuwirken.

7. Überwachungsverfahren nach einem der Ansprüche 2 bis 6, **dadurch gekennzeichnet, dass** es außerdem einen Schritt umfasst, bei dem die Korrekturmaßnahme in Abhängigkeit von einer Abweichung zwischen der vom ersten Sensor gemessenen Bakterienkonzentration im Wasser und dem variablen Momentanwert gesteuert wird.

8. Vorrichtung (10, 100) zur Überwachung der Konzentration von Bakterien im Wasser eines Wasserverteilungsnetzes (11), **dadurch gekennzeichnet, dass** sie Folgendes umfasst:
• einen ersten Sensor (12), der an einem ersten Ort (13) in dem Wasserverteilungsnetz (11) positioniert ist, der dazu bestimmt ist, eine Bakterienkonzentration (14) in dem Wasser an dem ersten Ort (13) zu messen,
• einen Rechner (15), der dazu bestimmt ist, einen einzigen variablen Momentanwert (16) der erwarteten Bakterienkonzentration im Wasser an der ersten Stelle (13) zu bestimmen, wobei dieser Wert sich entwickelt und regelmäßig aktualisiert wird, in Abhängigkeit von einem Parameter, der physikalisch-chemische und/oder bakteriologische Merkmale des Wassers umfasst,
• einen Komparator (17), der zum Vergleichen der vom ersten Sensor (12) gemessenen Bakterienkonzentration (14) im Wasser mit dem variablen Momentanwert (16) in einer vordefinierten Frequenz bestimmt ist.

9. Vorrichtung (10, 100) nach Anspruch 8, **dadurch gekennzeichnet, dass** sie eine Korrektureinheit (18) umfasst, die dazu bestimmt ist, auf die Konzentration von Bakterien im Wasser einzuwirken, wenn die vom ersten Sensor (12) gemessene Konzentration von Bakterien (14) im Wasser höher ist als der variable augenblickliche Wert (16).

10. Vorrichtung (10, 100) nach einem der Ansprüche 8 oder 9, **dadurch gekennzeichnet, dass** sie eine Datenbank (19) umfasst, die dazu bestimmt ist, die vom ersten Sensor (12) gemessene Konzentration von Bakterien (14) im Wasser abzuspeichern, um eine Historie der vom ersten Sensor (12) gemessenen Konzentrationen von Bakterien (14) im Wasser zu bilden, und dass der Rechner (15) so konfiguriert ist, dass er den Parameter, der physikalisch-chemische und/oder bakteriologische Merkmale des Wassers umfasst, auf der Grundlage der Historie der von dem ersten Sensor (12) gemessenen Konzentrationen von Bakterien in dem Wasser (14) bestimmt.

11. Überwachungsvorrichtung (10, 100) nach einem der Ansprüche 8 bis 10, **dadurch gekennzeichnet, dass** sie eine Vorrichtung (20) zum Messen einer Vielzahl von Merkmalen des Wassers umfasst, und dass der Rechner (15) so konfiguriert ist, dass er den charakteristischen Parameter des Wassers in Abhängigkeit von mindestens einem aus der Vielzahl von gemessenen Merkmalen des Wassers bestimmt.

12. Überwachungsvorrichtung (100) nach einem der Ansprüche 8 bis 11, **dadurch gekennzeichnet, dass** sie einen zweiten Sensor (22) umfasst, der an einem zweiten Ort (23) in dem Wasserverteilungsnetz positioniert ist, der dazu bestimmt ist, eine Konzentration von Bakterien (24) in dem Wasser an dem zweiten Ort (23) zu messen, und dass der Rechner (15) konfiguriert ist, um den Parameter, der physikalisch-chemische und/oder bakteriologische Merkmale des Wassers umfasst, in Abhängigkeit von der Konzentration von Bakterien in dem Wasser (24), die von dem zweiten Sensor (22) gemessen wurde, zu bestimmen.

13. Überwachungsvorrichtung (10, 100) nach einem der Ansprüche 9 bis 12, **dadurch gekennzeichnet, dass** die Korrektureinheit (18) eine Vorrichtung (30) zur Injektion eines Produkts umfasst, das in der Lage ist, dem Wachstum von Bakterien entgegenzuwirken.

14. Überwachungsvorrichtung (10, 100) nach einem der Ansprüche 9 bis 13, **dadurch gekennzeichnet, dass** sie eine Steuerung der Korrektureinheit (18) in Abhängigkeit von einer Abweichung zwischen der vom ersten Sensor (12) gemessenen Bakterienkonzentration im Wasser (14) und dem variablen Momentanwert (16) umfasst.

## Claims

1. A process for monitoring the concentration of bacteria in the water of a water distribution network under surrounding conditions, **characterized in that** it comprises the following steps:
• measuring the concentration of bacteria in the water by means of a first sensor positioned at a first location in the water distribution network (step 1001),
• determining a single variable instantaneous value of the expected concentration of bacteria in the water at the first location, an evolving and regularly updated value, as a function of a parameter comprising physico-chemical and/or bacteriological characteristics of the water, said parameter being evolving and taking account of the surrounding conditions of the water network (step 1002),
• at a predefined frequency: comparing the concentration of bacteria in the water measured by the first sensor to the variable instantaneous value (step 1003).

2. The monitoring process according to claim 1, **characterized in that** it further comprises a step (1004) of corrective action acting on the concentration of bacteria in the water if the concentration of bacteria in the water measured by the first sensor exceeds the variable instantaneous value.

3. The monitoring process according to any one of claims 1 or 2, **characterized in that** it comprises a step (1005) of saving the concentration of bacteria in the water measured by the first sensor in a database such as to constitute a log of the concentrations of bacteria in the water measured by the first sensor, and **in that** the parameter characteristic of the water is determined as a function of the log of the concentrations of bacteria in the water measured by the first sensor.

4. The monitoring process according to any one of the preceding claims, **characterized in that** it comprises a step (1006) of measuring a plurality of characteristics of the water, and **in that** the parameter characteristic of the water is determined as a function of at least one of the plurality of measured characteristics of the water.

5. The monitoring process according to any one of the preceding claims, **characterized in that** it further comprises a step (1007) of measuring the concentration of bacteria in the water measured by a second sensor positioned at a second location in the water distribution network, and **in that** the parameter characteristic of the water is determined as a function of the concentration of bacteria in the water measured by the second sensor.

6. The monitoring process according to any one of claims 2 to 5, **characterized in that** the corrective action (step 1004) comprises a step of injecting a product capable of countering the development of the bacteria.

7. The monitoring process according to any one of claims 2 to 6, **characterized in that** it further comprises a step of slaving the corrective action as a function of a difference between the concentration of bacteria in the water measured by the first sensor and the variable instantaneous value.

8. A device (10, 100) for monitoring the concentration of bacteria in the water of a water distribution network (11), **characterized in that** it comprises:
• a first sensor (12), positioned at a first location (13) in the water distribution network (11), intended to measure a concentration (14) of bacteria in the water at the first location (13),
• a calculator (15) intended to determine a unique variable instantaneous value (16) of the expected concentration of bacteria in the water at the first location (13), an evolving and regularly updated value, as a function of a parameter comprising physico-chemical and/or bacteriological characteristics of the water,
• a comparator (17) intended to compare the concentration (14) of bacteria in the water measured by the first sensor (12) to the variable instantaneous value (16) at a predefined frequency.

9. The device (10, 100) according to claim 8, **characterized in that** it comprises a correction unit (18) intended to act on the concentration of bacteria in the water if the concentration (14) of bacteria in the water measured by the first sensor (12) is greater than the variable instantaneous value (16).

10. The device (10, 100) according to any one of claims 8 or 9, **characterized in that** it comprises a database (19) intended to save the concentration (14) of bacteria in the water measured by the first sensor (12) such as to constitute a log of the concentrations (14) of bacteria in the water measured by the first sensor (12), and **in that** the calculator (15) is configured to determine the parameter comprising physico-chemical and/or bacteriological characteristics of the water as a function of the log of the concentrations (14) of bacteria in the water measured by the first sensor (12).

11. The monitoring device (10, 100) according to any one of claims 8 to 10, **characterized in that** it comprises a device (20) for measuring a plurality of characteristics of the water, and **in that** the calculator (15) is configured to determine the parameter characteristic of the water as a function of at least one of the plurality of measured characteristics of the water.

12. The monitoring device (100) according to any one of claims 8 to 11, **characterized in that** it comprises a second sensor (22), positioned at a second location (23) in the water distribution network, intended to measure a concentration (24) of bacteria in the water at the second location (23), and **in that** the calculator (15) is configured to determine the parameter comprising physico-chemical and/or bacteriological characteristics of the water as a function of the concentration (24) of bacteria in the water measured by the second sensor (22).

13. The monitoring device (10, 100) according to any one of claims 9 to 12, **characterized in that** the correction unit (18) comprises a device (30) for injecting a product capable of countering the development of the bacteria.

14. The monitoring device (10, 100) according to any one of claims 9 to 13, **characterized in that** it comprises a slaving of the correction unit (18) as a function of a difference between the concentration (14) of bacteria in the water measured by the first sensor (12) and the variable instantaneous value (16).
